# EUROPÄISCHE PATENTSCHRIFT

(11) **EP 1 372 608 B1**
(45) Veröffentlichungstag und Bekanntmachung des Hinweises auf die Patenterteilung: **10.10.2007**
(21) Anmeldenummer: 02701145.1
(22) Anmeldetag: 11.03.2002
(51) Int. Cl.: A61K 9/12, A61K 9/00, A61K 47/12

(54) **MEDIZINISCHE AEROSOLFORMULIERUNGEN**
MEDICAL AEROSOL FORMULATIONS
FORMULATIONS AEROSOL MEDICALES

(30) Priorität: 30.03.2001 CH 601012001; 20.08.2001 CH 152701
(43) Veröffentlichungstag der Anmeldung: 02.01.2004
(73) Patentinhaber: JAGOTEC AG, 4132 Muttenz (CH)
(72) Erfinder: MÜLLER-WALZ, Rudi, 79650 Schopfheim (DE); NIEDERLÄNDER, Carsten, CH-4125 Riehen (CH)
(74) Vertreter: Hoechst, Bruno Werner
(86) Internationale Anmeldenummer: PCT/CH2002/000145
(87) Internationale Veröffentlichungsnummer: WO 2002/078671

(56) Entgegenhaltungen:
- WO-A-98/33479

## Beschreibung

Die vorliegende Erfindung betrifft medizinische Suspensionsaerosolformulierungen sowie die Verwendung gewisser Salze als Hilfsstoffe in solchen Formulierungen.

Zur Herstellung medizinischer Dosieraerosole eignen sich in der Regel nur Treibgase, die sich bei Raumtemperatur verflüssigen lassen. In der Vergangenheit wurden üblicherweise Fluorchlorkohlenwasserstoffe (FCKWs), wie Trichlormonofluormethan (F11), Dichlordifluormethan (F12) und 1,2-Dichlor-1,1,2,2-tetrafluorethan (F114), und gelegentlich auch kurzkettige Alkane, wie z.B. Propan, Butan und Isobutan, verwendet.

Aufgrund der Ozonproblematik, hervorgerufen durch die Abspaltung von radikalischen Chloratomen aus den FCKWs, haben sich im Montrealer Abkomen viele Staaten darauf verständigt, die FCKWs als Treibmittel zukünftig nicht mehr zu verwenden. Als FCKW-Ersatzstoffe eignen sich im medizinischen Bereich fluorierte Alkane, insbesondere Hydrofluoralkane (im Rahmen der vorliegenden Erfindung auch als "HFA" bezeichnet) wie 1,1,1,2-Tetrafluorethan (HFA 134a) und 1,1,1,2,3,3,3-Heptafluorpropan (HFA 227), da sie inert sind und eine sehr geringe Toxizität aufweisen. Aufgrund ihrer physikalischen Eigenschaften, wie Druck, Dichte etc., sind letztere besonders geeignet, um FCKWs wie F11, F12 und F114 als Treibmittel in Dosieraerosolen zu ersetzen.

Es ist allgemein bekannt, dass im Falle von Suspensionsformulierungen nur Wirkstoffteilchen, die kleiner als etwa 6 µm sind, lungengängig sind. Zur gewünschten Deposition der Wirkstoffe in der Lunge müssen diese deshalb vor der Verarbeitung mittels spezieller Verfahren, wie z.B. Stift-, Kugel- oder Luftstrahlmühlen zerkleinert bzw. mikronisiert werden. Ein Mahlprozess führt aber zu einer Oberflächenvergrösserung, die in der Regel mit einer Erhöhung der elektrostatischen Ladung des mikronisierten Wirkstoffes einhergeht, wodurch dann meistens das Fliessverhalten und die Wirkstoff-Dispergierung verschlechtert wird. Als Folge der Grenzflächenaktivitäten kommt es häufig zu einer Agglomeration von Wirkstoffpartikeln oder auch zur Adsorption von Wirkstoff an Grenzflächen, die z.B. in der Anlagerung an Geräte oder Behälteroberflächen augenfällig wird.

Bei Aerosolzubereitungen, in denen der Wirkstoff in verflüssigtem Treibgas suspendiert vorliegt, kann es zu einer Deposition bzw. Ringbildung im Behälter an der Stelle kommen, wo die Flüssigphase in die Gasphase übergeht. Ohne Benetzung der mikronisierten Wirkstoffpartikel oder Abführen von Ladungen sowie Modifikation ihrer Oberflächeneigenschaften lassen sich Suspensionen nur unzureichend stabilisieren bzw. in dispergiertem Zustand halten. Die mangelhafte Benetzung bzw. Dispergierung der Wirkstoffpartikel hat auch zur Folge, dass diese in vielen Fällen eine hohe Adsorptionstendenz aufweisen und an Oberflächen wie der Behälterinnenwand oder dem Ventil kleben, was zu einer Unterdosierung sowie einer schlechten Dosiergenauigkeit von Sprühstoss zu Sprühstoss führt. Suspensionsformulierungen muss deshalb in der Regel ein oberflächenaktiver Hilfsstoff zugesetzt werden, um die Adsorption an Grenzflächen zu erniedrigen und eine akzeptable Dosiergenauigkeit zu erreichen. Besonders problematisch ist eine im Laufe der Lagerung eintretende Veränderung, insbesondere eine Erniedrigung des Anteils der inhalierbaren, lungengängigen Teilchen, der sogenannten Fine Particle Dose (FPD), was zu einer Abnahme der Wirksamkeit der Aerosolformulierung führt.

Zur Überwindung dieser Probleme werden in der Regel zugelassene oberflächenaktive Substanzen zugesetzt, wie sie bereits früher bei FCKW-haltigen Formulierungen Anwendung fanden, und in der Flüssigphase gelöst. Es hat sich jedoch gezeigt, dass die üblichen, in FCKW-haltigen Dosieraerosolen verwendeten Hilfsstoffe, wie Lecithin, Sorbitantrioleat und Ölsäure, in Hydrofluoralkanen, wie HFA 134 und HFA 227, nur unzureichend löslich sind. In JP 55-361 B wurden auch FCKW-haltige Aerosolformulierungen beschrieben, die als Suspendierhilfsmittel ein Metallsalz einer Fettsäure, beispielsweise ein Calcium- oder Aluminiumstearat, Magnesiumoleat oder Zinkisostearat, zusammen mit einem öllöslichen Lösungmittels, wie Isostearinsäure, 2-Octyldodecanol, 2-Hexadecanol, Isopropylmyristat, Trioleylphosphat Diethylenglycol, Diethylether und dergleichen, enthalten, um das Metallsalz in Lösung zu bringen. Solche Formulierungen haben sich jedoch in der Praxis nicht durchgesetzt.

Es wurde daher vorgeschlagen, die oberflächenaktiven Hilfsstoffe in HFA-haltigen Formulierungen nach Möglichkeit wegzulassen oder - falls sie aus technologischen Gründen unverzichtbar sind - ein polares Cosolvens wie z.B. Ethanol zuzusetzen, um in an sich bekannter Weise die Löslichkeit zu verbessern und die oberflächenaktiven Mittel in Lösung zu bringen. Andere Lösungsvorschläge beinhalten, die Wirkstoffteilchen mit dem oberflächenaktiven Mittel zu überziehen oder spezielle, treibgaslösliche oberflächaktive Mittel zu verwenden. Solche Vorschläge finden sich beispielsweise in US-A-2 868 691, US-A-3 014 844, DE-A-2 736 500, EP-A-0 372 777, WO-A-91/11495, EP-A-0 504 112, EP-B-0 550 031, WO-A-91/04011, EP-A-0 504 112 und WO-A-92/00061. In US-A-5 676 931 wurde für Formulierungen von LHRH-Analogen oder 5-Lipoxygenase-Inhibitoren vorgeschlagen, dem Wirkstoff/Treibgasgemisch einen als "Schutzkolloide" bezeichneten Hilfsstoff, vorzugsweise Cholesterol, Natriumlaurylsulfat, Stearinsäure, Caprylsäure oder Taurocholsäure, zuzusetzen. In WO-A-96/19198 wurden ferner pharmazeutische Aerosolformulierungen beschrieben, die neben einem Treibgas und einem zur Inhalation geeigneten Wirkstoff ein oberflächenaktives Mittel, ausgewählt aus C₈-C₁₆-Fettsäuren oder Salzen davon, Gallensäuresalzen, Phospholipiden und Alkylsacchariden, und gegebenenfalls bis zu 30 Gew.-% an Ethanol enthalten, wobei Gallensäuresalze bevorzugt und Beispiele nur für Natriumtaurocholat angegeben sind.

WO 98 33479 beschreibt Aerosol formulierungen, die unter anderen als Treibmittel methoxy-nonafluorbutan und calcium strearat enthalten.

Werden Cosolventien wie Ethanol in höherer Konzentration zugesetzt, erniedrigt sich allerdings die Dichte der Treibgasmischung, was vor allem bei Suspensionen zu einer unerwünschten Entmischung führen kann. Zudem kann man unerwünschterweise einen "nassen Spray" erhalten, weil das Treibgas viel schneller verdampft als Ethanol. Das ist unter anderem deshalb besonders nachteilig, weil bei Ethanolkonzentrationen von z.B. 10% oder mehr auf Grund der andersgearteten Verdampfungseigenschaften von Ethanol zum Treibgas vermehrt Teilchen mit grösserem aerodynamischem Durchmesser erzeugt werden und der Anteil inhalierbarer Partikel (< 6 µm) abnimmt. Als Folge davon kommt es zu einer Erniedrigung der für die Wirksamkeit entscheidenden Fine Particle Dose (FPD).

Daneben kann es durch die Erhöhung der Löslichkeit während der Lagerung auch zu Anlösungseffekten kommen, was zu Kristallwachstum und wiederum zu einer Erniedrigung der Menge an inhalierbaren, lungengängigen Teilchen, der sogenannten Fine Particle Dose (FPD), führt. Bei ethanolhaltigen Aerosolen kann es zudem gelegentlich zu Problemen der Wirkstoffstabilität kommen, insbesondere wenn der Wirkstoff in gelöster Form vorliegt.

Dies alles mag erklären, weshalb die meisten handelsüblichen Dosieraerosole als Suspensionen formuliert wurden.

Zur Messung der aerodynamischen Partikelgrössenverteilung bzw. der FPD oder der Fine Particle Fraction (FPF) eignen sich Impaktoren, wie z.B. der 5-Stufen Multistage Liquid Impinger (MSLI) oder 8-Stufen Andersen Kaskaden Impaktor (ACI), die in Chapter <601> der United States Pharmacopoeia (USP) oder in der Inhalanda Monographie der Europäischen Pharmakopöe (Ph. Eur.) beschrieben sind. Anhand -der aerodynamischen Partikelverteilung kann man mittels eines sogenannten "Log-probability plots" (logarithmische Darstellung der Wahrscheinlichkeitsverteilung) den mittleren aerodynamischen Teilchendurchmesser (Mass Median Aerodynamic Diameter MMAD) von Aerosol-Zubereitungen berechnen. Mit diesen Informationen zur Partikelverteilung erhält man Informationen, ob der Wirkstoff eher im oberen oder unteren Lungenbereich deponiert wird.

Wie sich aus dem Vorangehenden ergibt, stellt die Einhaltung einer hinreichend guten Dosiergenauigkeit, d.h. die gleichbleibende Wirkstofffreigabe von Sprühstoss zu Sprühstoss, ein grundsätzliches Problem der Suspensions-Dosieraerosole dar, das durch den Ersatz der FCKWs noch zusätzlich erschwert wird. Die Dosiergenauigkeit hängt neben dem Ventil und Adapter im wesentlichen von den Suspensionseigenschaften ab, d.h. davon, wie gut und homogen der Wirkstoff im Treibmittel dispergiert ist und wie lange die Suspension ohne Änderung ihrer physikalischen Eigenschaften in diesem labilen Gleichgewichtszustand verbleibt. Als besonders schwierig erweist sich die Einhaltung einer akzeptablen Dosiergenauigkeit im Falle potenter, niedrig dosierter Wirkstoffe. Beispielsweise benötigt man für den langwirkenden Beta-Agonisten Formoterol-fumarat, der bereits in sehr geringen Dosen (6 µg/Hub) therapeutisch wirksam ist, eine Formulierung, die eine hinreichend stabile Suspension ergibt, die nicht an Grenzflächen klebt und sich im Laufe der Lagerung bei unterschiedlichen Temperatur- und Feuchtebedingungen nicht verändert. Ein Überblick der am Markt befindlichen Produkte zeigt, dass es bis heute kein Dosieraerosol gibt, das Wirkstoffe in Mengen von weniger als 10 µg pro Hub (d.h. pro Sprühstoss) mit einer Streuung besser als ± 25% dosieren kann.

Der Erfindung liegt daher die Aufgabe zugrunde, die erwähnten Probleme von Suspensions-Dosieraerosolen möglichst weitgehend zu vermeiden und medizinische Suspensionsaerosolformulierungen bereitzustellen, die verbesserte Suspensions- und Haltbarkeitseigenschaften aufweisen und eine gute Dosiergenauigkeit - selbst bei niedrig dosierten Wirkstoffen - gestatten.

Die Aufgabe wird erfindungsgemäss gelöst durch Verwendung eines Carbonsäuresalzes, ausgewählt aus Calcium-; Magnesium- und Zinksalzen von Palmitin- und Stearinsäure, als fester Hilfsstoff in medizinischen Suspensionsaerosolformulierungen. Es wurde nämlich überraschenderweise gefunden, dass sich diese Salze als Suspendierhilfsmittel für medizinische Aerosolformulierungen eignen, obwohl sie in den gebräuchlichen Treibmitteln schlecht löslich sind. Ferner wurde überraschenderweise gefunden, dass diese Salze zugleich die Ventilfunktion verbessern, d.h. als Ventilschmiermittel wirken. In dieser Funktion bewirken die genannten Salze eine leichtgängigere, reibungslosere Betätigung der Ventile ohne übermässige Geräuschentwicklung und erhöhen die Dosiergenauigkeit. Überraschenderweise wurde weiterhin gefunden, dass sie auch die chemische Stabilität des pharmazeutischen Wirkstoffes, insbesondere die Feuchtigkeitsbeständigkeit feuchtigkeitsempfindlicher Wirkstoffe, verbessern können. Die Verwendung dieser Salze ermöglicht somit die Herstellung verbesserter Suspensionsaerosolformulierungen.

Die Erfindung betrifft daher die Verwendung eines Carbonsäuresalzes, ausgewählt aus Calcium-, Magnesium- und Zinksalzen von Palmitin- und Stearinsäure, als fester Hilfsstoff in medizinischen Suspensionsaerosolformulierungen zur Inhalation, umfassend ein druckverflüssigtes, nicht toxisches Treibmittel der allgemeinen Formel

CₓH_{y}F_{z} (I)

worin x für die Zahl 1, 2 oder 3 steht, y und z je
eine ganze Zahl ≥ 1 bedeuten und y + z = 2x + 2 ist, und einen im Treibmittel suspendierten, fein verteilten pharmazeutischen Wirkstoff, und insbesondere die Verwendung eines solchen Salzes zur Verbesserung der Suspensionsstabilität medizinischer Suspensionsaerosolformulierungen, zur Verbesserung der Dosiergenauigkeit von Druckgaspackungen medizinischer Suspensionsaerosolformulierungen, zur Verbesserung der Ventilfunktion des Dosierventils von Druckgaspackungen und/oder zur Verbesserung der chemischen Stabilität, insbesondere der Feuchtigkeitsbeständigkeit, pharmazeutischer Wirkstoffe in medizinischen Suspensionsaerosolformulierungen. Besonders vorteilhaft ist der Einsatz der erfindungsgemäss verwendbaren Palmitin- und Stearinsäuresalze in Aerosolformulierungen, die einen fein verteilten, durch Inhalation verabreichbaren pharmazeutischen Wirkstoff und als Hydrofluoralkan der (I) 1,1,1,2-Tetrafluorethan (HFA 134a) und/oder 1,1,1,2,3,3,3-Heptafluorpropan (HFA 227) enthalten. Dadurch lassen sich - wie nachfolgend beschrieben - insbesondere verbesserte Supensionsaerosolformulierungen für Wirkstoffe wie Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid, Salbutamol, Terbutalin und pharmazeutisch annehmbaren Salzen und Derivaten davon erhalten.

Die Erfindung betrifft ferner eine medizinische Aerosolformulierung zur Inhalation, umfassend ein druckverflüssigtes, nicht toxisches Treibmittel der allgemeinen Formel

CₓH_{y}F_{z} (I)

worin x für die Zahl 1, 2 oder 3 steht, y und z je
eine ganze Zahl ≥ 1 bedeuten und y + z = 2x + 2 ist, eine wirksame Menge eines im Treibmittel fein verteilten, suspendierten pharmazeutischen Wirkstoffes und einen festen Hilfsstoff, ausgewählt aus Calcium-, Magnesium- und Zinksalzen von Palmitin- und Stearinsäure. Gemäss einem bevorzugten Aspekt betrifft die Erfindung insbesondere eine medizinische Aerosolformulierung, umfassend
(a) ein druckverflüssigtes, nicht toxisches Treibmittel, ausgewählt aus 1,1,1,2-Tetrafluorethan, 1,1,1,2,3,3,3-Heptafluorpropan und Gemischen davon,
(b) eine wirksame Menge eines im Treibmittel fein verteilten, suspendierten pharmazeutischen Wirkstoffes; ausgewählt aus Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid, Salbutamol, Terbutalin und pharmazeutisch annehmbaren Salzen und Derivaten davon, und
(c) einen festen Hilfsstoff, ausgewählt aus Calcium-, Magnesium- und Zinksalzen von Palmitin- und Stearinsäure. Die Formulierung eignet sich insbesondere als Dosieraerosol für Druckgaspackungen.

Weiterhin betrifft die Erfindung die Herstellung der erfindungsgemässen Aerosolformulierung sowie eine Druckgaspackung, umfassend die erfindungsgemässe Aerosolformulierung in einem drucksicheren, mit einem Dosierventil versehenen Behälter.

Die Calcium-, Magnesium- und Zinksalze von Palmitin- und Stearinsäure sind seifenartige Verbindungen, die in druckverflüssigten Hydrofluoralkanen oder anderen Treibmitteln selbst bei Zusatz üblicher Cosolventien wie Ethanol schwer löslich und in der Regel praktisch unlöslich sind. Überraschenderweise wurde jedoch gefunden, dass der Einsatz dieser Salze in fester Form die Suspendierung pharmazeutischer Wirkstoffe in Hydrofluoralkanen und anderen Treibmitteln erleichtert und dass dadurch insbesondere medizinische Dosieraerosole mit verbesserten qualitätsrelevanten Eigenschaften, wie verbesserter Suspensionsstabilität, höherer Dosiergenauigkeit etc., erhalten werden können. Ein öllösliches Lösungsmittel, um den Hilfsstoff in der Formulierung zu lösen, ist erfindungsgemäss nicht erforderlich und sogar unerwünscht. Dieser Befund ist umso überraschender, als in GB-PS 837 465 und US-A-3 014 844 der Einsatz dispergierbarer oberflächenaktiver Hilfsstoffe in FCKW-Treibgasen bereits diskutiert, aber im Hinblick auf eine Verstopfung des Ventils und Adapters als ungeeignet beurteilt wurde, und in JP 55-361 B ein öllösliches Lösungsmittel zugesetzt werden musste, um Fettsäuresalze zu lösen.

Wird ein pharmazeutischer Wirkstoff, wie Formoterolfumarat, Levalbuterolsulfat und dergleichen, mit einem der erfindungsgemäss verwendbaren Suspendierhilfsmittel gemischt, erhält man ein Pulvergemisch, das sich in den üblichen Treibmitteln in der Regel auch in Abwesenheit von gelösten oberflächenaktiven Mitteln gut suspendieren lässt. Die erhaltenen Suspensionen können zudem selbst im Falle sehr niedriger Wirkstoffkonzentrationen genau dosiert werden, was möglicherweise auf die Bildung von Hilfsstoff-Wirkstoff-Assoziaten zurückzuführen sein könnte. Aufgrund dieser Eigenschaften eignen sich die erfindungsgemäss verwendbaren Hilfsstoffe daher unter anderem zur Verbesserung der Dosiergenauigkeit von Suspensionsformulierungen und insbesondere als Trägermaterialien zur Verdünnung niedrigdosierter Wirkstoffe zwecks Verbesserung der Dosiergenauigkeit.

Des weiteren wurde gefunden, dass die Adhäsionstendenz von elektrostatisch aufgeladenen Wirkstoffen durch Zumischen der erfindungsgemäss verwendbaren Hilfsstoffe reduziert wird, wodurch ihre Dispergierbarkeit verbessert wird.

Ferner wurde überraschenderweise gefunden, dass der Einsatz der erfindungsgemäss verwendbaren Hilfsstoffe die mechanische Funktion der Dosierventile verbessert. Obwohl diese Hilfsstoffe in den Treibmitteln in der Regel praktisch unlöslich sind und daher in suspendierter Form vorliegen, wirken sie aufgrund ihrer oberflächenaktiven Eigenschaften offenbar als Gleitmittel und verbessern dadurch die Ventilfunktion. Die gleichmässigere mechanische Funktion der Ventile führt in der Folge zu einer konsistenteren Dosierung des zu verabreichenden Dosieraerosols und damit ebenfalls zu einer Verbesserung der Dosiergenauigkeit.

Weiterhin wurde gefunden, dass der Einsatz der erfindungsgemäss verwendbaren Hilfsstoffe die chemische Stabilität, insbesondere die Feuchtigkeitsbeständigkeit, in der Formulierung vorhandener pharmazeutischer Wirkstoffe, wie Formoterolfumarat, Formoteroltartrat, Fenoterolhydrobromid, Salbutamolsulfat, Salbutamolacetat, Levalbuterolsulfat, Terbutalinsulfat, Tiotropiumbromid, Budesonid, Mometason, Fluticason und dergleichen, und damit auch die chemische Stabilität der Aerosolformulierung verbessert.

Die erfindungsgemäss verwendbaren Hilfsstoffe Magnesiumstearat, Magnesiumpalmitat, Calciumstearat, Calciumpalmitat, Zinkstearat und Zinkpalmitat erlauben daher die Herstellung verbesserter Suspensionsaerosolformulierungen und, gewünschtenfalls, den Verzicht auf die üblicherweise verwendeten oberflächenaktiven Mittel (Ölsäure, Sorbitantrioleat und Lecithin), die in Hydrofluoralkanen nur unter Einsatz eines Cosolvens weiter verwendbar sind. Als erfindungsgemäss verwendbare Stearate eignen sich insbesondere auch handelsübliche Stearate, die bis zu etwa einen Drittel an entsprechendem Palmitat enthalten können. Besonders bevorzugt sind Magnesiumstearat und Gemische von Magnesiumstearat und Magnesiumpalmitat.

Die erfindungsgemässe Aerosolformulierung kann den pharmazeutischen Wirkstoff gewünschtenfalls in Form eines pharmazeutisch annehmbaren Salzes oder Derivates, wie z.B. Formoterolfumarat, Formoteroltartrat, Salmeterolxinafoat, Fenoterol-Hydrobromid, Clenbuterol-Hydrochlorid, Levalbuterolsulfat, Ipratropiumbromid, Oxitropiumbromid, Glycopyrroniumbromid, Tiotropiumbromid, Mometasonfuroat, Fluticasondipropionat, Beclomethasondipropionat, Flunisolidacetat, Salbutamolsulfat, Salbutamolacetat oder Terbutalinsulfat, enthalten. Wirkstoffe mit chiralen Zentren können in Form ihres wirksamen Enantiomers oder als Enantiomerengemisch (z.B. Racemat) verwendet werden. Gewünschtenfalls können die erfindungsgemässen Aerosolformulierungen auch zwei oder mehrere pharmazeutisch Wirkstoffe enthalten, wobei Kombinationen von Fluticason, Ipratropium, Oxitropium, Glycopyrronium, Tiotropium, Budesonid, Mometason, Ciclesonid, Rofleponid oder einem pharmazeutisch annehmbaren Salz oder Derivat davon mit Salbutamol, Levalbuterol, Fenoterol, Terbutalin, Formoterol und/ oder Salmeterol oder einem pharmazeutisch annehmbaren Salz oder Derivat davon bevorzugt sind. Gewünschtenfalls können die erfindungsgemässen Aerosolformulierungen neben einem oder mehreren suspendierten Wirkstoffen auch gelöste pharmazeutische Wirkstoffe enthalten.

Der Gehalt an pharmazeutischem Wirkstoff in den erfindungsgemässen Aerosolformulierungen ist nicht kritisch und wird in der Regel vor allem von der gewünschten, therapeutisch oder prophylaktisch wirksamen Dosis und damit von der Wirksamkeit des jeweiligen Wirkstoffes abhängen. Beispielsweise kann der Gehalt an suspendiertem pharmazeutischem Wirkstoff etwa 0,0001 bis 5 Gew.-% oder mehr, vorzugsweise etwa 0,001 bis 2 Gew.-%, bezogen auf die Gesamtformulierung, betragen. Da die Vorteile der erfindungsgemässen Aerosolformulierung bei hochwirksamen, d.h. niedrig dosierten Wirkstoffen besonders ausgeprägt sind, ist sie für Formulierungen mit vergleichsweise niedrigen Wirkstoffkonzentrationen von beispielsweise etwa 0,0001 bis 0,4 Gew.-%, 0,001 bis 0,1 Gew.-% oder 0,001 bis 0,04 Gew.-% besonders geeignet. Da die Hubmassen handelsüblicher MDIs (Metered Dose Inhalers) meist im Bereich von etwa 30 bis 130 mg (mit Ventilen entsprechend etwa 25 bis 100 µl) und typischerweise bei etwa 70 mg liegen, lassen sich somit mit den erfindungsgemässen Formulierungen insbesondere auch Dosen von etwa 0,1 bis 100 µg, 0,1 bis 50 µg oder 0,1 bis 20 µg an pharmazeutischem Wirkstoff pro Sprühstoss verabreichen.

Der zu suspendierende Wirkstoff bzw. die zu suspendierenden Wirkstoffe können in an sich bekannter Weise, z.B. mittels Stift-, Kugel- oder Luftstrahlmühlen, mikronisiert oder durch kontrollierte Mikrokristallisation oder Fällung erhalten und im Treibmittel suspendiert werden. Um eine möglichst vollständige Inhalierbarkeit zu gewährleisten und zu vermeiden, dass kleine Teilchen wieder ausgeatmet werden, besitzen die suspendierten Wirkstoffteilchen vorzugsweise einen mittleren aerodynamischen Teilchendurchmesser MMAD (Mass Median Aerodynamic Diameter, Massenmittel) im Bereich von etwa 1 bis 6 µm, beispielsweise etwa 2 bis 5 µm.

Die erfindungsgemäss verwendbaren Hilfsstoffe sind dem Fachmann bekannt und im Handel erhältlich oder in bekannter Weise aus den Carbonsäuren herstellbar; beispielsweise werden Erdalkali-, Aluminium- und Zinksalze langkettiger Carbonsäuren gelegentlich als Hilfsstoffe bei der Herstellung von Wasser-in-öl-Emulsionen verwendet werden. Der Ausdruck "festes Salz" bzw. "fester Hilfsstoff" umfasst im Rahmen der vorliegenden Erfindung insbesondere solche Salze bzw. Hilfsstoffe, die bei 20°C in kristalliner oder amorpher Form vorliegen können, wobei solche bevorzugt sind, die bei etwa 50°C oder 60°C noch in kristalliner oder amorpher Form vorliegen können. Selbstverständlich sind auch Hilfsstoffe geeignet, die sowohl kristalline als auch amorphe Anteile enthalten. Erfindungsgemäss eignen sich - wie oben erwähnt - insbesondere auch handelsübliche Formen der Hilfsstoffe, wie beispielsweise handelsübliches Magnesiumstearat, das typischerweise bis zu etwa einen Drittel an Magnesiumpalmitat enthalten kann.

Die Teilchengrösse des erfindungsgemäss verwendeten Hilfsstoffes ist nicht kritisch. Gewünschtenfalls kann der Hilfsstoff ebenfalls in mikronisierter Form mit einem mittleren aerodynamischen Teilchendurchmesser MMAD von etwa 1 bis 6 µm, beispielsweise etwa 2 bis 5 µm, eingesetzt werden, insbesondere wenn die gleichzeitige Inhalation des Hilfsstoffes erwünscht ist. Die Mikronisierung kann in an sich bekannter Weise nach den oben im Zusammenhang mit dem Wirkstoff erwähnten Methoden erfolgen. Hingegen wird vorzugsweise Hilfsstoff mit einem mittleren aerodynamischen Teilchendurchmesser MMAD von mehr als 6 µm, beispielsweise etwa 10 bis 100 µm, verwendet, wenn erwünscht ist, dass der Hilfsstoff nicht in die Lunge gelangt.

Der Anteil an festem Suspendierhilfsstoff in den erfindungsgemässen Formulierungen kann in einem relativ breiten Bereich variieren, wobei meist schon geringe Mengen ausreichend sind, um die gewünschten Verbesserungen zu erzielen. Typischerweise kann das Gewichtsverhältnis zwischen suspendiertem pharmazeutischem Wirkstoff und Hilfsstoff etwa 50:1 bis etwa 1:10 betragen, wobei ein Bereich von etwa 10:1 bis etwa 1:5 meist bevorzugt ist. Bezogen auf die Gesamtformulierung kann der Anteil an festem Hilfsstoff typischerweise etwa 1 Gew.-% oder weniger, beispielsweise etwa 0,0001 bis 1 Gew.-%, betragen; höhere Mengen sind jedoch in der Regel nicht nachteilig. Im allgemeinen sind aber Mengen von etwa 0,005 bis 0,5 Gew.-%, insbesondere etwa 0,01 bis 0,2 Gew.-%, bezogen auf die Gesamtformulierung, bevorzugt, insbesondere wenn der Wirkstoff ebenfalls in niedriger Konzentration vorliegt. Der Hilfsstoffanteil pro Sprühstoss beträgt daher im allgemeinen nicht mehr als etwa 500 µg und liegt meist im Bereich von etwa 5 bis 250 µg bzw. 10 bis 100 µg.

Vorzugsweise kann der Hilfsstoff je nach verwendetem Wirkstoff und Treibmittel so ausgewählt werden, dass die Dichte der suspendierten Materialien insgesamt der Dichte des Treibmittels möglichst weitgehend angepasst ist. Beispielsweise kann mikronisiertes Formoterolfumarat, das in HFA 227 zum Flotieren neigt, mit Magnesiumstearat, das zum Sedimentieren neigt, kombiniert werden, um das suspendierte Material besser in Schwebe zu halten und Flotation oder Sedimentation zu minimieren, womit die physikalische Stabilität der Suspension weiter verbessert wird.

HFA 134a und HFA 227 besitzen bei 20°C einen Dampfdruck von ca. 6 bar bzw. ca. 4,2 bar. Diese beiden Treibgase unterscheiden sich hinsichtlich ihrer Dichte (ca. 1,2 g/ml für HFA 134a und ca. 1,4 g/ml für HFA 227), was insofern von Bedeutung ist, als durch geeignete Wahl des Treibmittels oder Treibmittelgemisches dessen Dichte besser an die Dichte der suspendierten Substanzen angeglichen und damit letztere besser in Schwebe gehalten werden können. Gewünschtenfalls kann die Dichte des Treibmittels auch durch Zusatz von Cosolventien oder anderen Treibgasen, wie beispielsweise Ethanol, Diethylether, Propan, n-Butan, Isobutan und dergleichen, weiter erniedrigt werden. Im Hinblick auf die Ozonproblematik werden aber vorzugsweise keine oder nur geringe Mengen an FCKWs verwendet.

In den erfindungsgemässen Aerosolformulierungen kann der Anteil an 1,1,1,2-Tetrafluorethan (HFA 134a) und/oder 1,1,1,2,3,3,3-Heptafluorpropan (HFA 227), bezogen auf die Gesamtformulierung, vorzugsweise mindestens etwa 50 Gew.-% und besonders bevorzugt mindestens etwa 80 Gew.-% betragen. In der Regel ist es vorteilhaft, wenn das Treibmittel ausschliesslich aus HFA 134a und/oder HFA 227 besteht oder deren Anteil an der Gesamtformulierung 90 Gew.-% oder mehr beträgt.

Gewünschtenfalls können die erfindungsgemässen Aerosolformulierungen als weiteres Treibgas Stickstoff oder insbesondere Distickstoffmonoxid (Lachgas) und/oder Kohlendioxid in einer Menge von etwa 0,0001 bis 10 Gew.-% enthalten. Konzentrationen von etwa 0,01 bis 3 Gew.-% sind im allgemeinen bevorzugt und Konzentrationen von etwa 0,1 bis 1,0 Gew.-% besonders bevorzugt; höhere Konzentrationen sind in der Regel nur dann sinnvoll, wenn die Formulierung einen vergleichsweise hohen Anteil an Cosolvens enthält. Wie in WO-A-98/34595 und WO-A-00/06121 gefunden wurde, können nämlich Treibgase mit vorteilhafteren Eigenschaften erhalten werden, wenn man den üblichen Treibgasen, insbesondere den genannten Hydrofluoralkanen, eine geringe Menge an Distickstoffmonoxid und/oder Kohlendioxid zusetzt. Derartige Treibgasgemische zeigen - im Unterschied zu Distickstoffmonoxid und Kohlendioxid als alleinige Treibgase - bei zunehmender Entleerung nur eine geringfügige Abnahme des Binnendrucks im Behältnis, was deren Verwendung als Treibmittel für Dosieraerosole ermöglicht. Zudem wurde festgestellt, dass die Zugabe von Distickstoffmonoxid und/oder Kohlendioxid die Suspendierung von pharmazeutischen Wirkstoffen erleichtert, womit eher auf die Zugabe von oberflächenaktiven Stoffen und/oder Cosolventien verzichtet oder zumindest deren Anteil verringert werden kann. Des weiteren wurde gefunden, dass durch Zugabe von Distickstoffmonoxid und/oder Kohlendioxid die unerwünschte Deposition von Wirkstoff im Oropharynx reduziert und gleichzeitig die Fine Particle Dose erhöht werden kann. Ferner kann man durch Zugabe dieser Treibgase Sauerstoff aus den Hydrofluoralkanen oder anderen Treibmitteln verdrängen, was die Lagerstabilität von oxidationsempfindlichen Wirkstoffen verbessert, und je nach Menge an Distickstoffmonoxid und/oder Kohlendioxid den Binnendruck im Aerosolbehältnis so einstellen, wie es für die jeweilige Anwendung am sinnvollsten ist.

Die erfindungsgemässen Aerosolformulierungen besitzen bei 20°C vorzugsweise einen Druck von etwa 3 bis 10 bar, insbesondere etwa 3,5 bis 6 bar. Ein allenfalls niedrigerer Druck kann vorzugsweise durch Zugabe von Distickstoffmonoxid und/oder Kohlendioxid entsprechend erhöht werden.

Die vorliegende Erfindung erlaubt in der Regel den völligen Verzicht auf Cosolventien und herkömmliche, im Treibmittel oder Treibmittel/Cosolvens-Gemisch lösliche oberflächenaktive Mittel. Insbesondere kann die erfindungsgemässe Aerosolformulierung im wesentlichen frei von im Treibmittel oder Treibmittel/Cosolvens-Gemisch löslichen, d.h. vollständig gelösten, oberflächenaktiven Mitteln sein, wobei der Ausdruck "im wesentlichen frei" vorzugsweise einen Gehalt von weniger als 0,0001 Gew.-%, bezogen auf die Gesamtformulierung, bedeutet. Gewünschtenfalls ist jedoch die Weiterverwendung von üblichen oberflächenaktiven Mitteln, wie Ölsäure, Lecithin, Sorbitantrioleat und dergleichen, nicht ausgeschlossen.

Die Zugabe einer geringen Menge an Cosolvens kann hingegen gelegentlich von Vorteil sein. Als Cosolventien eignen sich beispielsweise Wasser, Alkohole mit 1 bis 3 Kohlenstoffatomen, Alkane mit 3 bis 6 Kohlenstoffatome, Dialkylether mit 2 bis 4 Kohlenstoffatomen und dergleichen. Beispiele geeigneter Cosolventien sind: Ethanol, Propanol, Isopropanol, Ethylenglykol, Propylenglykol, Glycerin, Propan, Butan, Isobutan, Pentan, Dimethylether und Diethylether, wobei Ethanol, Ethylenglykol, Glycerin, Propylenglykol und Diethylether oder deren Gemische und insbesondere Ethanol in der Regel bevorzugt sind. Im allgemeinen liegt jedoch der Anteil an Cosolventien wie Ethanol, falls vorhanden, nicht über etwa 15 Gew.-%, beispielsweise im Bereich von etwa 0,1 bis 15 Gew.-%, vorzugsweise aber nicht über etwa 10 Gew.-% und meist nicht über etwa 5 Gew.-%, bezogen auf die Gesamtformulierung.

Weiterhin können die erfindungsgemässen Aerosolformulierungen gewünschtenfalls Puffersubstanzen oder Stabilisatoren wie Citronensäure, Ascorbinsäure, Natrium-EDTA, Vitamin E, N-Acetylcystein und dergleichen enthalten. Im allgemeinen werden solche Substanzen, falls vorhanden, in Mengen von nicht mehr als etwa 1 Gew.-%, beispielsweise in einer Menge von etwa 0,0001 bis 1 Gew.-%, bezogen auf die Gesamtformulierung, verwendet.

Im allgemeinen sind jedoch Aerosolformulierungen bevorzugt, die aus den oben genannten Komponenten (a), (b) und (c) bestehen oder zusätzlich als Cosolvens Ethanol und/oder zusätzlich als weiteres Treibgas Distickstoffmonoxid und/oder Kohlendioxid enthalten. Ein bevorzugter Aspekt der Erfindung betrifft daher medizinische Aerosolformulierungen, bestehend aus
(a) einem druckverflüssigten, nicht toxischen Treibmittel, ausgewählt aus 1,1,1,2-Tetrafluorethan, 1,1,1,2,3,3,3-Heptafluorpropan und Gemischen davon,
(b) einer wirksamen Menge mindestens eines im Treibmittel fein verteilten, suspendierten pharmazeutischen Wirkstoffes, ausgewählt aus Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid, Salbutamol, Terbutalin und pharmazeutisch annehmbaren Salzen und Derivaten davon,
(c) einem festen Hilfsstoff, ausgewählt aus Calcium-, Magnesium- und Zinksalzen von Palmitin- und Stearinsäure,
(d) gegebenenfalls Distickstoffmonoxid und/oder Kohlendioxid in einer Menge von 0,0001 bis 10 Gew.-%, vorzugsweise 0,01 bis 3 Gew.-%, bezogen auf die Gesamtformulierung, und
(e) gegebenenfalls Ethanol.

Gemäss einem bevorzugten Aspekt kann diese Formulierung als Wirkstoff Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid oder ein pharmazeutisch annehmbares Salz oder Derivat eines dieser Wirkstoffe enthalten, wobei Formulierungen von Formoterol, Salmeterol, Fenoterol, Levalbuterol, Oxitropium, Tiotropium, Budesonid, Mometason, Fluticason und von pharmazeutisch annehmbaren Salzen oder Derivaten dieser Wirkstoffe besonders bevorzugt sind. Gemäss einem weiteren bevorzugten Aspekt kann die vorangehend definierte Formulierung als Wirkstoff Salbutamol, Terbutalin oder ein pharmazeutisch annehmbares Salz oder Derivat eines dieser Wirkstoffe enthalten.

Als Beispiele besonders bevorzugter erfindungsgemässer Aerosolformulierungen können die folgenden genannt werden, in denen die Komponenten jeweils in den oben angegebenen Mengen vorliegen können und in denen sich aber insbesondere die nachfolgend als bevorzugt genannten Komponenten und Mengen als vorteilhaft erwiesen haben:
- Aerosolformulierung, bestehend aus Budesonid, mindestens einem Treibmittel, ausgewählt aus HFA 134a und HFA 227, mindestens einem Hilfsstoff, ausgewählt aus Calciumpalmitat, Calciumstearat, Magnesiumpalmitat, Magnesiumstearat, Zinkpalmitat und Zinkstearat, gegebenenfalls einem zusätzlichen Treibgas, ausgewählt aus Distickstoffmonoxid und Kohlendioxid, und gegebenenfalls bis zu 0,5 Gew.-% Ethanol; vorzugsweise kann die Formulierung aus 0,1-1,0 Gew.-% Budesonid, 0,005-0,2 Gew.-% Hilfsstoff, 0-1 Gew.-% Distickstoffmonoxid und/oder Kohlendioxid, 0-0,5 Gew.-% Ethanol und aus HFA 134a und/ oder HFA 227 (Rest) bestehen; vorzugsweise kann der Hilfsstoff Magnesiumstearat oder ein Gemisch von Magnesiumstearat und Magnesiumpalmitat sein; das Treibmittel ist vorzugsweise HFA 134a oder ein Gemisch von HFA 134a und HFA 227; besonders bevorzugt sind Formulierungen, die aus Budesonid, HFA 134a und erfindungsgemässem Hilfsstoff, umfassend Magnesiumstearat, bestehen;
- Aerosolformulierung, bestehend aus einem Beta-Agonisten, ausgewählt aus Formoterol, Fenoterol, Salbutamol, Salmeterol, Levalbuterol, Terbutalin und pharmazeutisch annehmbaren Derivaten und Salzen davon, mindestens einem Treibmittel, ausgewählt aus HFA 134a und HFA 227, mindestens einem Hilfsstoff, ausgewählt aus Calciumpalmitat, Calciumstearat, Magnesiumpalmitat, Magnesiumstearat, Zinkpalmitat und Zinkstearat, gegebenenfalls einem zusätzlichen Treibgas, ausgewählt aus Distickstoffmonoxid und Kohlendioxid, und gegebenenfalls Ethanol; vorzugsweise kann die Formulierung aus 0,001-0,1 Gew.-% Beta-Agonist, 0,0001-0,2 Gew.-% Hilfsstoff, 0-1 Gew.-% Distickstoffmonoxid und/oder Kohlendioxid, 0,1-10 Gew.-% Ethanol und aus HFA 134a und/oder HFA 227 (Rest) bestehen; vorzugsweise kann der Hilfsstoff Magnesiumstearat oder ein Gemisch von Magnesiumstearat und Magnesiumpalmitat sein; das Treibmittel ist vorzugsweise HFA 227 oder ein Gemisch von HFA 134a und HFA 227; besonders bevorzugt sind Formulierungen, die als Wirkstoff Formoterol oder ein pharmazeutisch annehmbares Salz oder Derivat davon, insbesondere Formoterolfumarat oder Formoteroltartrat, enthalten; ebenfalls besonders bevorzugt sind Formulierungen, die als Wirkstoff Salbutamol oder ein pharmazeutisch annehmbares Salz oder Derivat davon, insbesondere Salbutamolsulfat oder Salbutamolacetat, enthalten;
- Aerosolformulierung, bestehend aus Budesonid, einem Beta-Agonisten, ausgewählt aus Formoterol, Fenoterol, Salbutamol, Salmeterol, Levalbuterol, Terbutalin und pharmazeutisch annehmbaren Derivaten und Salzen davon, mindestens einem Treibmittel, ausgewählt aus HFA 134a und HFA 227, mindestens einem Hilfsstoff, ausgewählt aus Calciumpalmitat, Calciumstearat, Magnesiumpalmitat, Magnesiumstearat, Zinkpalmitat und Zinkstearat, gegebenenfalls einem zusätzlichen Treibgas, ausgewählt aus Distickstoffmonoxid und Kohlendioxid, und gegebenenfalls bis zu 0,5 Gew.-% Ethanol; vorzugsweise kann die Formulierung aus 0,1-1,0 Gew.-% Budesonid, 0,001-2 Gew.-% (insbesondere 0,001-0,04 Gew.-%) Beta-Agonist, 0,005-0,2 Gew.-% Hilfsstoff, 0-1 Gew.-% Distickstoffmonoxid und/oder Kohlendioxid, 0-0,5 Gew.-% Ethanol und aus HFA 134a und/oder HFA 227 (Rest) bestehen; vorzugsweise kann der Hilfsstoff Magnesiumstearat oder ein Gemisch von Magnesiumstearat und Magnesiumpalmitat sein; vorzugsweise kann die Formulierung frei von Ethanol sein; besonders bevorzugt sind Formulierungen, in denen der Beta-Agonist Formoterol oder ein pharmazeutisch annehmbares Salz oder Derivat davon, insbesondere Formoterolfumarat oder Formoteroltartrat, und das Treibmittel HFA 134a oder ein Gemisch von HFA 134a und HFA 227, z.B. ein Gemisch im Gewichtsverhältnis von etwa 70:30, ist;
- Aerosolformulierung, bestehend aus Fluticason oder einem pharmazeutisch annehmbaren Salz oder Derivat (vorzugsweise Fluticasondipropionat) davon, einem Beta-Agonisten, ausgewählt aus Formoterol, Fenoterol, Salbutamol, Salmeterol, Levalbuterol, Terbutalin und pharmazeutisch annehmbaren Derivaten und Salzen davon, mindestens einem Treibmittel, ausgewählt aus HFA 134a und HFA 227, mindestens einem Hilfsstoff, ausgewählt aus Calciumpalmitat, Calciumstearat, Magnesiumpalmitat, Magnesiumstearat, Zinkpalmitat und Zinkstearat, gegebenenfalls einem zusätzlichen Treibgas, ausgewählt aus Distickstoffmonoxid und Kohlendioxid, und gegebenenfalls bis zu 10 Gew.-% Ethanol; vorzugsweise kann die Formulierung aus 0,1-1,0 Gew.-% Fluticason oder Salz oder Derivat davon, 0,001-2 Gew.-% (insbesondere 0,001-0,04 Gew.-%) Beta-Agonist, 0,005-0,2 Gew.-% Hilfsstoff, 0-1 Gew.-% Distickstoffmonoxid und/oder Kohlendioxid, 0,1-10 Gew.-% Ethanol und aus HFA 134a und/oder HFA 227 (Rest) bestehen; vorzugsweise.kann der Hilfsstoff Magnesiumstearat oder ein Gemisch von Magnesiumstearat und Magnesiumpalmitat sein;
- Aerosolformulierung, bestehend aus Fluticason oder einem pharmazeutisch annehmbaren Salz oder Derivat davon (vorzugsweise Fluticasondipropionat), mindestens einem Treibmittel, ausgewählt aus HFA 134a und HFA 227, mindestens einem Hilfsstoff, ausgewählt aus Calciumpalmitat, Calciumstearat, Magnesiumpalmitat, Magnesiumstearat, Zinkpalmitat und Zinkstearat, und gegebenenfalls einem zusätzlichen Treibgas, ausgewählt aus Distickstoffmonoxid und Kohlendioxid; vorzugsweise kann die Formulierung aus 0,1-1,0 Gew.-% Fluticason oder dessen Derivat, 0,005-0,5 Gew.-% Hilfsstoff, 0-1 Gew.-% (z.B. 0,1-1,0 Gew.-%) Distickstoffmonoxid und/oder Kohlendioxid und aus HFA 134a und/oder HFA 227 (Rest) bestehen; vorzugsweise kann der Hilfsstoff Zinkstearat oder ein Gemisch von Zinkstearat und Zinkpalmitat sein; das Treibmittel ist vorzugsweise HFA 227 oder ein Gemisch von HFA 134a und HFA 227.

Die Herstellung der erfindungsgemässen Aerosolformulierungen kann in an sich bekannter Weise dadurch erfolgen, dass man den mikronisierten pharmazeutischen Wirkstoff und den Hilfsstoff in das druckverflüssigte Treibmittel einbringt. Die Formulierungen können unter Verwendung von üblichen Rührern und Homogenisatoren hergestellt werden. Zur Abfüllung können bekannte Verfahren, wie die Kalt- oder Druckfülltechnik oder Modifikationen dieser Techniken, eingesetzt werden. Als Behältnisse eignen sich beispielsweise drucksichere Behälter aus Glas, Kunststoff oder Aluminium, die mit Dosierventilen von z.B. 10 bis 140 µl bestückt und mit handelsüblichen - auch atemzuggetriggerten - Mundrohradaptern versehen werden können.

Die vorliegende Erfindung ermöglicht somit die Herstellung von Dosieraerosolen mit vorteilhafteren Eigenschaften, wie anhand der nachfolgenden Beispiele weiter veranschaulicht wird. In den Beispielen bedeutet der Ausdruck "mikronisiert" jeweils, dass das betreffende Material einen mittleren aerodynamischen Teilchendurchmesser von weniger als 6 µm aufweist.

### Beispiel 1

24,96 g mikronisiertes Budesonid und 3,12 g Magnesiumstearat werden in einen Druckansatzkessel eingewogen. Nach Verschliessen und Evakuieren des Ansatzkessels werden unter Rühren 7,8 kg HFA 134a zugegeben. Nach dem Homogenisieren wird die erhaltene Suspension mittels Druckfülltechnik in mit Dosierventilen verschlossene Aluminiumdosen abgefüllt.

Die abgefüllte Suspension zeichnet sich gegenüber einer mit gleichen Mengen Budesonid und HFA 134a, aber ohne Magnesiumstearat-Zusatz hergestellten Suspension durch ein grösseres Flockenvolumen und eine längere Schwebezeit der suspendierten Bestandteile aus. Mit handelsüblichen Dosierventilen ergibt die erfindungsgemässe Suspension eine bessere Dosiergenauigkeit von Hub zu Hub. Weiterhin zeigt die erfindungsgemässe Suspension eine deutlich verbesserte Ventilgängigkeit, während das Ventil bei der Vergleichsformulierung ohne Magnesiumstearat bei Aktivierung deutlich stärker beansprucht wird (Reibgeräusche), was im Extremfall zu Undichtigkeit im Ventil führt.

### Beispiel 2

1,09 g mikronisiertes Formoterolfumarat und 0,182 g Magnesiumstearat werden in einen Druckansatzkessel eingewogen. Nach Verschliessen und Evakuieren des Ansatzkessels werden 12,4 kg HFA 227 zugegeben, das zuvor in einem anderen Druckansatzkessel mit 0,4 kg Ethanol versetzt wurde. Nach dem Homogenisieren dieser Mischung wird die erhaltene Suspension mittels Druckfülltechnik in mit Dosierventilen verschlossene Aluminiumdosen abgefüllt.

### Beispiel 3

21,22 g mikronisiertes Budesonid und 0,54 g Magnesiumstearat werden in einen Druckansatzkessel eingewogen. Nach Verschliessen und Evakuieren des Ansatzkessels werden 6,24 kg eines Treibgasgemisches von HFA 227 und HFA 134a (Gewichtsverhältnis 30:70) zugegeben, das zuvor in einem anderen Druckansatzkessel mit 0,002 Gew.-% Ethanol versetzt wurde. Nach dem Homogenisieren dieser Mischung wird die erhaltene Suspension in einen anderen Druckansatzkessel überführt, in den zuvor 0.64 g Formoterol-fumarat eingewogen wurden. Die Suspension wird nochmals homogenisiert und mittels Druckfülltechnik in mit Dosierventilen verschlossene Aluminiumdosen abgefüllt.

### Beispiel 4

11,2 g mikronisiertes Glycopyrroniumbromid und 1,1 g Magnesiumstearat werden in einen Druckansatzkessel eingewogen. Nach Verschliessen und Evakuieren des Ansatzkessels werden unter Rühren 14 kg eines Treibgasgemisches von HFA 227 und HFA 134a (Gewichtsverhältnis 50:50) zugegeben, das zuvor in einem anderen Druckansatzkessel mit 1,4 Gew.-% Ethanol versetzt wurde. Nach dem Homogenisieren wird die erhaltene Suspension mittels Druckfülltechnik in mit Dosierventilen verschlossene Aluminiumdosen abgefüllt.

### Beispiel 5

32 g mikronisiertes Fluticasondipropionat und 3,9 g Zinkstearat werden in einen Druckansatzkessel eingewogen. Nach Verschliessen und Evakuieren des Ansatzkessels werden unter Rühren 9,75 kg HFA 227 zugegeben, das zuvor in einem anderen Druckansatzkessel mit Distickstoffoxid begast und auf einen Druck von 5 bar bei 20°C eingestellt wurde. Nach dem Homogenisieren wird die erhaltene Suspension mittels Druckfülltechnik in mit Dosierventilen verschlossene Aluminiumdosen abgefüllt.

### Beispiel 6

14,4 g mikronisiertes Ipratropiumbromid und 21,6 g Calciumstearat werden in einen Druckansatzkessel eingewogen. Nach Verschliessen und Evakuieren des Ansatzkessels werden unter Rühren 50,4 kg HFA 227 zugegeben, das zuvor in einem anderen Druckansatzkessel mit Distickstoffoxid begast und auf einen Druck von 5 bar bei 20°C eingestellt wurde. Nach dem Homogenisieren wird die erhaltene Suspension mittels Druckfülltechnik in mit Dosierventilen verschlossene Aluminiumdosen abgefüllt.

## Patentansprüche

1. Medizinische Aerosolformulierung zur Inhalation, umfassend ein druckverflüssigtes, nicht toxisches Treibmittel der allgemeinen Formel
CₓHyF_{z} (I)
worin x für die Zahl 1, 2 oder 3 steht, y und z je
eine ganze Zahl ≥ 1 bedeuten und y + z = 2x + 2 ist, eine wirksame Menge eines im Treibmittel fein verteilten, suspendierten pharmazeutischen Wirkstoffes und einen festen Hilfsstoff, ausgewählt aus Calcium-, Magnesium- und Zinksalzen von Palmitin- und Stearinsäure.

2. Aerosolformulierung nach Anspruch 1, worin das Treibmittel 1,1,1,2-Tetrafluorethan, 1,1,1,2,3,3,3-Heptafluorpropan oder ein Gemisch der beiden umfasst.

3. Aerosolformulierung nach Anspruch 1 oder 2, umfassend
(a) ein druckverflüssigtes, nicht toxisches Treibmittel, ausgewählt aus 1,1,1,2-Tetrafluorethan, 1,1,1,2,3,3,3-Heptafluorpropan und Gemischen davon,
(b) eine wirksame Menge eines im Treibmittel fein verteilten, suspendierten pharmazeutischen Wirkstoffes, ausgewählt aus Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid, Salbutamol, Terbutalin und pharmazeutisch annehmbaren Salzen und Derivaten davon, und
(c) einen festen Hilfsstoff, ausgewählt aus Calcium-, Magnesium- und Zinksalzen von Palmitin- und Stearinsäure.

4. Aerosolformulierung nach einem der Ansprüche 1 bis 3, bestehend aus
(a) einem druckverflüssigten, nicht toxischen Treibmittel, ausgewählt aus 1,1,1,2-Tetrafluorethan, 1,1,1,2,3,3,3-Heptafluorpropan und Gemischen davon,
(b) einer wirksamen Menge mindestens eines im Treibmittel fein verteilten, suspendierten pharmazeutischen Wirkstoffes, ausgewählt aus Formoterol, Salmeterol, Fenoterol, Clenbuterol, Levalbuterol, Ipratropium, Oxitropium, Glycopyrronium, Tiotropium, Budesonid, Ciclesonid, Mometason, Fluticason, Beclomethason, Flunisolid, Loteprednol, Triamcinolon, Amilorid, Rofleponid, Salbutamol, Terbutalin und pharmazeutisch annehmbaren Salzen und Derivaten davon,
(c) einem festen Hilfsstoff, ausgewählt aus Calcium-, Magnesium- und Zinksalzen von Palmitin- und Stearinsäure,
(d) gegebenenfalls einem zusätzlichen Treibmittel, ausgewählt aus Distickstoffmonoxid und Kohlendioxid, in einer Menge von 0,0001 bis 10 Gew.-%, bezogen auf die Gesamtformulierung, und
(e) gegebenenfalls Ethanol.

5. Aerosolformulierung nach einem der Ansprüche 2 bis 4, worin 1,1,1,2-Tetrafluorethan, 1,1,1,2,3,3,3-Heptafluorpropan oder ein Gemisch der beiden in einer Menge von mindestens 50 Gew.-%, bezogen auf die Gesamtformulierung, vorliegt.

6. Aerosolformulierung nach einem der Ansprüche 2 bis 5, worin 1,1,1,2-Tetrafluorethan, 1,1,1,2,3,3,3-Heptafluorpropan oder ein Gemisch der beiden in einer Menge von mindestens 80 Gew.-%, bezogen auf die Gesamtformulierung, vorliegt.

7. Aerosolformulierung nach einem der Ansprüche 1 bis 6, worin der Hilfsstoff in einer Menge von 0,0001 bis 1 Gew.-%, bezogen auf die Gesamtformulierung, vorliegt.

8. Aerosolformulierung nach einem der Ansprüche 1 bis 7, worin der Hilfsstoff in einer Menge von 0,005 bis 0,5 Gew.-%, bezogen auf die Gesamtformulierung, vorliegt.

9. Aerosolformulierung nach einem der Ansprüche 1 bis 8, worin der Hilfsstoff in einer Menge von 0,01 bis 0,2 Gew.-%, bezogen auf die Gesamtformulierung, vorliegt.

10. Aerosolformulierung nach einem der Ansprüche 1 bis 9, worin der suspendierte pharmazeutische Wirkstoff in einer Menge von 0,0001 bis 5 Gew.-%, bezogen auf die Gesamtformulierung, vorliegt.

11. Aerosolformulierung nach einem der Ansprüche 1 bis 10, bestehend aus
(a) einem druckverflüssigten, nicht toxischen Treibmittel, ausgewählt aus 1,1,1,2-Tetrafluorethan, 1,1,1,2,3,3,3-Heptafluorpropan und Gemischen davon,
(b) einer wirksamen Menge Budesonid,
(c) einem festen Hilfsstoff, ausgewählt aus Calciumpalmitat, Calciumstearat, Magnesiumpalmitat, Magnesiumstearat, Zinkpalmitat und Zinkstearat,
(d) gegebenenfalls einem zusätzlichen Treibmittel, ausgewählt aus Distickstoffmonoxid und Kohlendioxid, in einer Menge von 0,0001 bis 10 Gew.-%, bezogen auf die Gesamtformulierung, und
(e) gegebenenfalls Ethanol in einer Menge von bis zu 0,5 Gew.-%, bezogen auf die Gesamtformulierung.

12. Aerosolformulierung nach Anspruch 11, **dadurch gekennzeichnet, dass** Budesonid in einer Menge von 0,1 bis 1 Gew.-% und der Hilfsstoff in einer Menge von 0,005 bis 0,2 Gew.-%, jeweils bezogen auf die Gesamtformulierung, vorliegen.

13. Aerosolformulierung nach Anspruch 11 oder 12, worin der Hilfsstoff Magnesiumstearat umfasst.

14. Verwendung eines Carbonsäuresalzes, ausgewählt aus Calcium-, Magnesium- und Zinksalzen von Palmitin- und Stearinsäure, als fester Hilfsstoff in medizinischen Suspensionsaerosolformulierungen zur Inhalation, umfassend ein druckverflüssigtes, nicht toxisches Treibmittel der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, und einen im Treibmittel suspendierten, fein verteilten pharmazeutischen Wirkstoff.

## Claims

1. Medical aerosol formulation for inhalation, comprising a pressure-liquefied, non-toxic propellant of the general formula
CₓH_{y}F_{z} (I)
wherein x is the number 1, 2 or 3,
y and z each denote an integer ≥ 1 and
y+z=2x+2,
an efficacious amount of a finely divided pharmaceutically active compound suspended in the propellant and a solid excipient, selected from calcium, magnesium and zinc salts of palmitic and stearic acid.

2. Aerosol formulation according to claim 1, wherein the propellant comprises 1,1,1,2-tetrafluoroethane, 1,1,1,2,3,3,3-heptafluoropropane or a mixture of the two.

3. Aerosol formulation according to claim 1 or 2, comprising
(a) a pressure-liquefied, non-toxic propellant, selected from 1,1,1,2-tetrafluoroethane, 1,1,1,2,3,3,3-heptafluoropropane and mixtures thereof,
(b) an efficacious amount of a finely divided pharmaceutically active compound suspended in the propellant, selected from formoterol, salmeterol, fenoterol, clenbuterol, levalbuterol, ipratropium, oxytropium, glycopyrronium, tiotropium, budesonide, ciclesonide, mometasone, fluticasone, beclomethasone, flunisolide, loteprednol, triamcinolone, amiloride, rofleponide, salbutamol, terbutaline and pharmaceutically acceptable salts and derivatives thereof, and
(c) a solid excipient, selected from calcium, magnesium and zinc salts of palmitic and stearic acid.

4. Aerosol formulations as claimed in one of claims 1 to 3, consisting of (a) a pressure-liquefied, non-toxic propellant, selected from 1,1,1,2-tetrafluoroethane, 1,1,1,2,3,3,3-hepta-fluoropropane and mixtures thereof,
(b) an efficacious amount of at least one finely divided pharmaceutically active compound suspended in the propellant, selected from formoterol, salmeterol, fenoterol, clenbuterol, levalbuterol, ipratropium, oxytropium, glycopyrronium, tiotropium, budesonide, ciclesonide, mometasone, fluticasone, beclomethasone, flunisolide, loteprednol, triamcinolone, amiloride, rofleponide, salbutamol, terbutaline and pharmaceutically acceptable salts and derivatives thereof, and
(c) a solid excipient, selected from calcium, magnesium and zinc salts of palmitic and stearic acid,
(d) optionally an additional propellant, selected from dinitrogen monoxide and carbon dioxide, in an amount of from 0.0001 to 10% by weight, based on the total formulation, and
(e) optionally ethanol.

5. Aerosol formulation according to one of claims 2 to 4, wherein 1,1,1,2-tetrafluoroethane, 1,1,1,2,3,3,3-heptafluoropropane or a mixture of the two is present in an amount of at least 50% by weight, based on the total formulation.

6. Aerosol formulation according to one of claims 2 to 5, wherein 1,1,1,2-tetrafluoroethane, 1,1,1,2,3,3,3-heptafluoropropane or a mixture of the two is present in an amount of at least 80% by weight, based on the total formulation.

7. Aerosol formulation according to one of claims 1 to 6, wherein the excipient is present in an amount of from 0.0001 to 1% by weight, based on the total formulation.

8. Aerosol formulation according to one of claims 1 to 7, wherein the excipient is present in an amount of 0.005 to 0.5% by weight, based on the total formulation.

9. Aerosol formulation according to one of claims 1 to 8, wherein the excipient is present in an amount of 0.01 to 0.2% by weight, based on the total formulation.

10. Aerosol formulation according to one of claims 1 to 9, wherein the suspended pharmaceutical active compound is present in an amount of from 0.0001 to 5% by weight, based on the total formulation.

11. Aerosol formulation according to one of claims 1 to 10, consisting of
(a) a pressure-liquefied, non-toxic propellant, selected from 1,1,1,2-tetrafluoroethane, 1,1,1,2,3,3,3-hepta-fluoropropane and mixtures thereof,
(b) an efficacious amount of budesonide,
(c) a solid excipient, selected from calcium palmitate, calcium stearate, magnesium palmitate, magnesium stearate, zinc palmitate and zinc stearate, (d) optionally an additional propellant, selected from dinitrogen monoxide and carbon dioxide, in an amount of from 0.0001 to 10% by weight, based on the total formulation, and
(e) optionally ethanol in an amount of up to 0.5% by weight, based on the total formulation.

12. Aerosol formulation according to claim 11, **characterized in that** budesonide is present in an amount of from 0.1 to 1% by weight and the excipient is present in an amount of from 0.005 to 0.2% by weight, in each case based on the total formulation.

13. Aerosol formulation according to claim 11 or 12, wherein the excipient comprises magnesium stearate.

14. Use of a carboxylic acid salt, selected from calcium, magnesium and zinc salts of palmitic and stearic acid, as a solid excipient in medical suspension aerosol formulations for inhalation, comprising a pressure-liquefied, non-toxic propellant of the general formula
CₓH_{y}F_{z} (I)
wherein x is the number 1, 2 or 3,
y and z each denote an integer ≥ 1 and
y+z = 2x+2,
and a finely divided pharmaceutically active compound suspended in the propellant.

## Revendications

1. Formulation aérosol médicale pour l'inhalation, comprenant un propulseur non toxique liquéfié sous pression, de formule générale
CₓH_{y}F_{z} (I)
dans laquelle x représente le nombre 1, 2 ou 3, y et z représentent chacun un
nombre entier ≥ 1 et y + z = 2x + 2,
une quantité efficace d'un principe actif pharmaceutique finement divisé en suspension dans le propulseur et un adjuvant solide choisi parmi les sels de calcium, magnésium et zinc d'acide palmitique et d'acide stéarique.

2. Formulation aérosol selon la revendication 1, dans laquelle le propulseur est le 1,1,1,2-tétrafluoroéthane, le 1,1,1,2,3,3,3-heptafluoropropane ou un mélange des deux.

3. Formulation aérosol selon la revendication 1 ou 2, comprenant
(a) un propulseur non toxique liquéfié sous pression, choisi parmi le 1,1,1,2-tétrafluoroéthane, le 1,1,1,2,3,3,3-heptafluoropropane ou des mélanges de ceux-ci,
(b) une quantité efficace d'un principe actif pharmaceutique finement divisé en suspension dans le propulseur, choisi parmi le formotérol, le salmétérol, le fénotérol, le clenbutérol, le lévalbutérol, l'ipratropium, l'oxitropium, le glycopyrronium, le tiotropium, le budésonide, le ciclésonide, la mométasone, la fluticasone, la béclométhasone, le flunisolide, le lotéprednol, la triamcinolone, l'amiloride, le rofléponide, la salbutamol, la terbutaline et leurs dérivés et sels pharmaceutiquement acceptables, et
(c) un adjuvant solide choisi parmi les sels de calcium, magnésium et zinc d'acide palmitique et d'acide stéarique.

4. Formulation aérosol selon l'une quelconque des revendications 1 à 3, constituée
(a) d'un propulseur non toxique liquéfié sous pression, choisi parmi le 1,1,1,2-tétrafluoroéthane, le 1,1,1,2,3,3,3-heptafluoropropane ou des mélanges de ceux-ci,
(b) d'une quantité efficace d'au moins un principe actif pharmaceutique finement divisé en suspension dans le propulseur, choisi parmi le formotérol, le salmétérol, le fénotérol, le clenbutérol, le lévalbutérol, l'ipratropium, l'oxitropium, le glycopyrronium, le tiotropium, le budésonide, le ciclésonide, la mométasone, la fluticasone, la béclométhasone, le flunisolide, le lotéprednol, la triamcinolone, l'amiloride, le rofléponide, la salbutamol, la terbutaline et leurs dérivés et sels pharmaceutiquement acceptables, et
(c) d'un adjuvant solide choisi parmi les sels de calcium, magnésium et zinc d'acide palmitique et d'acide stéarique,
(d) éventuellement d'un propulseur supplémentaire, choisi parmi le protoxyde d'azote et le dioxyde de carbone, en une proportion de 0,0001 à 10 % en poids, par rapport à la composition totale, et
(e) éventuellement d'éthanol.

5. Formulation aérosol selon l'une quelconque des revendications 2 à 4, dans laquelle le 1,1,1,2-tétrafluoroéthane, le 1,1,1,2,3,3,3-heptafluoropropane ou un mélange des deux est présent en une proportion d'au moins 50 % en poids, par rapport à la formulation totale.

6. Formulation aérosol selon l'une quelconque des revendications 2 à 5, dans laquelle le 1,1,1,2-tétrafluoroéthane, le 1,1,1,2,3,3,3-heptafluoropropane ou un mélange des deux est présent en une proportion d'au moins 80 % en poids, par rapport à la formulation totale.

7. Formulation aérosol selon l'une quelconque des revendications 1 à 6, dans laquelle l'adjuvant est présent en une proportion de 0,0001 à 1 % en poids, par rapport à la formulation totale.

8. Formulation aérosol selon l'une quelconque des revendications 1 à 7, dans laquelle l'adjuvant est présent en une proportion de 0,005 à 0,5 % en poids, par rapport à la formulation totale.

9. Formulation aérosol selon l'une quelconque des revendications 1 à 8, dans laquelle l'adjuvant est présent en une proportion de 0,01 à 0,2 % en poids, par rapport à la formulation totale.

10. Formulation aérosol selon l'une quelconque des revendications 1 à 9, dans laquelle le principe actif pharmaceutique en suspension est présent en une proportion de 0,0001 à 5 % en poids, par rapport à la formulation totale.

11. Formulation aérosol selon l'une quelconque des revendications 1 à 10, constituée
(a) d'un propulseur non toxique liquéfié sous pression, choisi parmi le 1,1,1,2-tétrafluoroéthane, le 1,1,1,2,3,3,3-heptafluoropropane ou des mélanges de ceux-ci,
(b) d'une quantité efficace de budésonide,
(c) d'un adjuvant solide choisi parmi le palmitate de calcium, le stéarate de calcium, le palmitate de magnésium, le stéarate de magnésium, le palmitate de zinc et le stéarate de zinc,
(d) éventuellement d'un propulseur supplémentaire, choisi parmi le protoxyde d'azote et le dioxyde de carbone, en une proportion de 0,0001 à 10 % en poids, par rapport à la composition totale, et
(e) éventuellement d'éthanol en une proportion allant jusqu'à 0,5 % en poids, par rapport à la formulation totale.

12. Formulation aérosol selon la revendication 11, **caractérisée en ce que** le budésonide est présent en une proportion de 0,1 à 1 % en poids et l'adjuvant est présent en une proportion de 0,005 à 0,2 % en poids, chaque fois par rapport à la formulation totale.

13. Formulation aérosol selon la revendication 11 ou 12, dans laquelle l'adjuvant comprend le stéarate de magnésium.

14. Utilisation d'un sel d'acide carboxylique, choisi parmi les sels de calcium, magnésium et zinc d'acide palmitique et d'acide stéarique, en tant qu'adjuvant solide dans des formulations aérosol médicales en suspension destinées à l'inhalation, comprenant un propulseur non toxique liquéfié sous pression, de formule générale
CₓH_{y}F_{z}
dans laquelle x représente le nombre 1, 2 ou 3, y et z représentent chacun un nombre entier ≥ 1 et y + z = 2x + 2,
et un principe actif pharmaceutique finement divisé en suspension dans le propulseur.
